# EUROPEAN PATENT APPLICATION

(11) **EP 2 005 949 A1**
(43) Date of publication of application: **24.12.2008**
(21) Application number: 07738128.3
(22) Date of filing: 09.03.2007
(51) Int. Cl.: A61K 31/05, A23L 1/16, A23L 1/30, A61K 45/00, A61P 9/00

(54) **CARDIAC DYSFUNCTION-AMELIORATING AGENT OR CARDIAC FUNCTION-MAINTAINING AGENT**

(30) Priority: 13.03.2006 JP 2006066992; 21.11.2006 JP 2006314034
(71) Applicant: Kaneka Corporation, Kita-ku Osaka-shi, Osaka 530-8288 (JP)
(72) Inventor: KISHIDA, Hideyuki, Kakogawa-shi, Hyogo 6750039 (JP); FUJII, Kenji, Kobe-shi, Hyogo 6511202 (JP); KUBO, Hiroshi, Kobe-shi, Hyogo 6550046 (JP); HOSOE, Kazunori, Takasago-shi, Hyogo 6760025 (JP)
(74) Representative: Gillet, Raphaëlle
(86) International application number: PCT/JP2007/054643
(87) International publication number: WO 2007/105621

(57) **Abstract**

An object of the present invention is to provide a highly safe oral composition superior in a cardiac dysfunction-ameliorating or cardiac function-maintaining action. The present inventors have conducted intensive studies in an attempt to solve the aforementioned problems and found that use of, particularly, reduced coenzyme Q10 from among highly safe coenzyme Q affords a composition useful for amelioration of cardiac dysfunction and maintenance of cardiac function. Accordingly, the present invention provides a cardiac dysfunction-ameliorating agent or cardiac function-maintaining agent containing reduced coenzyme Q as an active ingredient, and a pharmaceutical product, a food, an animal drug, a feed and the like, which contain the agent.

## Description

### Technical Field

The present invention relates to a cardiac dysfunction-ameliorating agent or cardiac function-maintaining agent comprising reduced coenzyme Q as an active ingredient.

### Background Art

Cardiac disease is one of the important causes of death for Japanese people, of which angina pectoris, myocardial infarction and cardiac failure are highly important as the pathology potentially directly causing death. "Easily-fatigued", "short breath", "episode of chest pain", "palpitation", "dizziness, faint" and the like are symptoms frequently associated with cardiac diseases. For example, "episode of chest pain" is a symptom mostly related to angina pectoris·myocardial infarction, and "easily-fatigued", "short breath" and "palpitation" are symptoms mostly related to cardiac failure. Angina pectoris is a state in which arteriosclerosis is formed in the coronary of the heart, the arteriosclerosis causes stenosis which prevents sufficient supply of blood to the cardiac muscle, and the cardiac muscle runs short of oxygen and produces a chest pain. Myocardial infarction refers to the pathology where the coronary is completely obstructed and blood supply is completely prevented, thus causing a partial death of the heart. Cardiac failure refers to the pathology where the hematological supply is insufficient for the systemic demand, since the pumping function of the heart became lower. Cardiac failure is the pathology that commonly occurs at late stages of any cardiac diseases, which shows characteristic symptoms of short breath, generalized fatigability, palpitation, swelling of lower leg and the like.

Angina pectoris, myocardial infarction and cardiac failure are treated by a drug therapy, a catheter treatment, a surgical treatment and the like. Particularly, in cardiac diseases, a drug therapy is highly important among the treatments. Therefore, many drugs are currently used for the treatment of cardiac diseases. Nevertheless, cardiac disease is the second major cause of death next to cancer for Japanese people, and the number thereof keeps rising due to the westernized eating habits and lifestyle. In the circumstances, there is a demand for the development of a pharmaceutical product or functional food showing an ameliorating or prophylactic action on cardiac dysfunction, which is safe and can be continuously ingested every day.

Such reports relating to a composition having a cardiac dysfunction-ameliorating or cardiac function-maintaining action concern a composition or food and drink containing, as an active ingredient, arachidonic acid or a compound containing arachidonic acid as a constituent fatty acid (patent reference 1), a composition containing pycnogenol (patent reference 2) and the like.

Coenzyme Q is an essential component widely distributed in living organisms from bacterium to mammal, and is known to be an electron transport system constituent component of mitochondria in the cells of a living body. Coenzyme Q functions as a transport component in the electron transport system by repeating oxidization and reduction in mitochondria, and is one of the coenzymes necessary for producing ATP (adenosine triphosphate), which is an intracorporeal energy source. In human, coenzyme Q10 in which the side chain of coenzyme Q has 10 repeat structures is the main component, and aids the life activity of cells and tissues. In Japan, oxidized coenzyme Q10 is used as a drug for congestive heart failure, and in Europe and the United States, oxidized coenzyme Q10 is widely used as a health food. There are reports on the effectiveness for cardiac diseases other than cardiac failure, such as angina pectoris (non-patent reference 1), myocardial infarction (non-patent reference 2) and the like, as well as hypertension (non-patent reference 3).

An important characteristic of coenzyme Q10 is its high safety. It has been reported that consecutive administration of 1200 mg/kg/day for 52 weeks in a chronic toxicity test of oxidized coenzyme Q10 in rats did not show a toxic influence (non-patent reference 4). For human (body weight 50 kg), 1200 mg/kg/day corresponds to 60 g/day. In view of the fact that the usual dosage of oxidized coenzyme Q10 used as a health food in Europe and the United States is 100-300 mg/day, coenzyme Q10 is an extremely highly safe supplement material.

It is known that generally about 40-90% of coenzyme Q in the living organisms is in a reduced form. It has also been reported that a pharmaceutical composition containing reduced coenzyme Q is superior in oral absorbability (patent reference 3). However, reduced coenzyme Q is difficult to handle since it is easily oxidized by oxygen in the air, and the oxidized coenzyme Q is generally conventionally commercially available and is used as a pharmaceutical agent or food because once ingested, oxidized coenzyme Q is converted to reduced coenzyme Q in the body. As mentioned above, the action of oxidized coenzyme Q on the cardiac function has already been confirmed and oxidized coenzyme Q has been put to practical use. Due to the aforementioned circumstances, however, application of reduced coenzyme Q itself to a cardiac function associated disease has not been tried. For example, there are reports on the methods for the treatment and/or prophylaxis of blood vessel diseases characterized by a shortage of nitric oxide (NO), comprising combining a therapeutic agent for angina pectoris and an antioxidant, which include a case reciting oxidized coenzyme Q and reduced coenzyme Q as simple examples of the antioxidant (patent reference 4), and a report on a composition that enhances cell energy metabolism by combining oxidized coenzyme Q10 or reduced coenzyme Q10 with two or more kinds of cytochromes a, b, c and the like (patent reference 5). Nevertheless, the action of reduced coenzyme Q itself on the cardiac function has not been confirmed.
patent reference 1: JP-A-2005-132758
patent reference 2: JP-A-2005-239581
patent reference 3: JP-A-10-109933
patent reference 4: National Publication of International Patent Application No. 2003-514020
patent reference 5: National Publication of International Patent Application No. 2004-518712
non-patent reference 1: Am J Cardiol 1985; 56(4): 247-51.
non-patent reference 2: Mol Cell Biochem 2003; 246(1-2): 75-82.
non-patent reference 3: Biofactors 2003; 18(1-4): 91-100.
non-patent reference 4: J Agric Food Chem 1999; 47: 3756-3763.

### Disclosure of the Invention

### Problems to be Solved by the Invention

An object of the present invention is to provide a highly safe composition superior in a cardiac dysfunction-ameliorating or cardiac function-maintaining action.

### Means of Solving the Problems

The present inventors have conducted intensive studies in an attempt to solve the aforementioned problems and found that use of, particularly, reduced coenzyme Q10 from among highly safe coenzyme Q is effective for the amelioration of cardiac dysfunction and maintenance of cardiac function. Accordingly, the present invention is as follows.
[1] A cardiac dysfunction-ameliorating agent or cardiac function-maintaining agent comprising reduced coenzyme Q represented by the following formula (1):

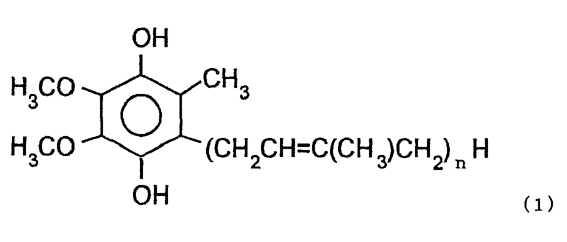

wherein n is an integer of 1-12, as an active ingredient.
[2] The cardiac dysfunction-ameliorating agent or cardiac function-maintaining agent of [1], wherein the reduced coenzyme Q is a reduced coenzyme Q10 wherein n is 10.
[3] The cardiac dysfunction-ameliorating agent or cardiac function-maintaining agent of [1] or [2], further comprising a pharmaceutical agent for treating a cardiac disease.
[4] The cardiac dysfunction-ameliorating agent or cardiac function-maintaining agent of [3], wherein the pharmaceutical agent for treating a cardiac disease is one or more kinds of pharmaceutical agents selected from the group consisting of a cardiotonic agent, an antiarrhythmic agent, a vasodilator, an antihypertensive agent, an antiplatelet agent and a diuretic.
[5] A pharmaceutical product or quasi-drug, comprising the cardiac dysfunction-ameliorating agent or cardiac function-maintaining agent of any of [1] to [4].
[6] A food comprising the cardiac dysfunction-ameliorating agent or cardiac function-maintaining agent of any of [1] to [4].
[7] The food of [6], which is a food with health claims.
[8] The food with health claims of [7], which is a food for specified health uses.
[9] An animal drug comprising the cardiac dysfunction-ameliorating agent or cardiac function-maintaining agent of any of [1] to [4].
[10] A feed for ameliorating or preventing cardiac dysfunction, comprising the cardiac dysfunction-ameliorating agent or cardiac function-maintaining agent of any of [1] to [4].
[11] A method of controlling cardiac function, comprising administering a cardiac dysfunction-ameliorating agent or cardiac function-maintaining agent comprising a reduced coenzyme Q represented by the following formula (1):

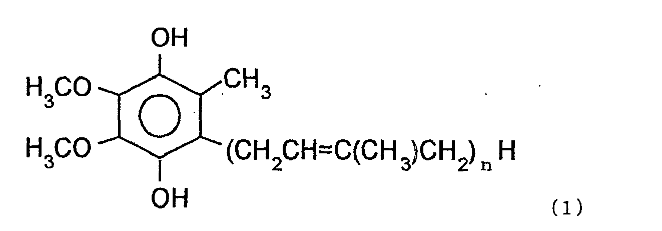

wherein n is an integer of 1-12, as an active ingredient, to a subject of administration.
[12] The method of [11], wherein the aforementioned subject of administration is a healthy human or healthy animal having a potential risk of cardiac dysfunction.
[13] The method of [11], wherein the aforementioned subject of administration is suffering from cardiac dysfunction and under a treatment thereof, and the method further comprises administering the aforementioned cardiac dysfunction-ameliorating agent or cardiac function-maintaining agent as an aid of the treatment.
[14] Use of a reduced coenzyme Q represented by the following formula (1):

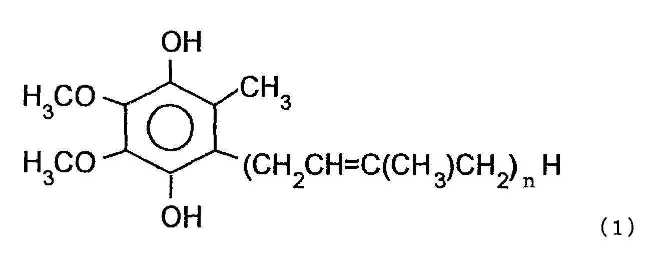

wherein n is an integer of 1-12, for the production of a cardiac dysfunction-ameliorating agent or cardiac function-maintaining agent.
[15] A commercial package comprising the cardiac dysfunction-ameliorating agent or cardiac function-maintaining agent of any of [1] to [4], and a written matter stating that the cardiac dysfunction-ameliorating agent or cardiac function-maintaining agent can or should be used for controlling cardiac function.

### Effect of the Invention

The cardiac dysfunction-ameliorating agent or cardiac function-maintaining agent of the present invention comprising reduced coenzyme Q10 as an active ingredient provides superior effects of ameliorated condition of cardiac dysfunction and maintenance of cardiac function. In addition, the cardiac dysfunction-ameliorating agent or cardiac function-maintaining agent of the present invention causes no side effects and is highly safe. Thus, a composition comprising the agent can be ingested daily and continuously not only as a pharmaceutical product but also as a quasi-drug, a food or a food with health claims.

### Best Mode for Carrying Out the Invention

The cardiac dysfunction-ameliorating agent or cardiac function-maintaining agent of the present invention comprises reduced coenzyme Q as an active ingredient. In the present invention, the cardiac dysfunction means a disease state where the heart function is impaired or does not work properly, such as angina pectoris, myocardial infarction, cardiac failure, cardiac muscle disease, valvular disease, arrhythmia and the like, and a pre-disease state associated with the risk thereof. The cardiac dysfunction-ameliorating agent or cardiac function-maintaining agent of the present invention has an action of ameliorating the condition of the cardiac dysfunction, or an action of preventing the condition, namely, an action of controlling cardiac function.

The cardiac dysfunction-ameliorating agent or cardiac function-maintaining agent of the present invention contains reduced coenzyme Q as an essential active ingredient. However, reduced coenzyme Q can be used alone, or coenzyme Q in a mixed form of reduced coenzyme Q and oxidized coenzyme Q can also be used. When coenzyme Q in a mixed form of reduced coenzyme Q and oxidized coenzyme Q is to be used, the proportion of reduced coenzyme Q relative to the total coenzyme Q is preferably not less than 20 wt%, more preferably not less than 40 wt%, further preferably not less than 50 wt%, still more preferably not less than 60 wt%, particularly preferably not less than 80 wt%, most preferably not less than 96 wt%, relative to the total amount of coenzyme Q (i.e., total amount of reduced coenzyme Q and oxidized coenzyme Q). While the upper limit of the proportion of reduced coenzyme Q relative to the total coenzyme Q is not particularly limited, it is generally not more than 99.9 wt%, preferably not more than 99.5 wt%. Use of reduced coenzyme Q alone (i.e., 100 wt% reduced type) is admissible.

The proportion of oxidized coenzyme Q and reduced coenzyme Q in coenzyme Q can be generally determined by a method including quantifying oxidized coenzyme Q and reduced coenzyme Q in a sample by an HPLC system using a UV detector and calculating the proportion based on the obtained amount ratio, or a method including calculating the proportion of oxidized coenzyme Q and reduced coenzyme Q from peak areas obtained by a system combining HPLC with an electrochemical detector. A system incorporating an electrochemical detector is highly useful for measuring the ratio of reduced type present in a trace amount in a living organism or sample, since it can specifically measure an oxidized or reduced substance and has high sensitivity. The present invention measured by incorporating an electrochemical detector manufactured by Shiseido Co., Ltd. into HPLC analysis apparatus manufactured by SHIMADZU Corporation under the conditions of the following HPLC.

column: YMC-Pack (ODS-A303), detection wavelength: 275 nm,
mobile phase: methanol (88%)+ hexane (12%), flow rate: 1 ml/min.

The method of obtaining reduced coenzyme Q to be used in the present invention is not particularly limited and, for example, a method including obtaining coenzyme Q containing oxidized coenzyme Q as a main component by a conventionally known method such as synthesis, fermentation, extraction from naturally occurring substances and the like, and concentrating reduced coenzyme Q fraction in an outflow fluid by chromatography, and the like can be employed. In this case, where necessary, a general reducing agent such as sodium borohydride, sodium dithonite (sodium hydrosulfite), ascorbic acid and the like is added to the above-mentioned coenzyme Q, oxidized coenzyme Q contained in the above-mentioned coenzyme Q is reduced according to a conventional method to give reduced coenzyme Q, and the reduced coenzyme Q is concentrated by chromatography. In addition, reduced coenzyme Q can also be obtained by a method including reacting an existing high-purity coenzyme Q (oxidized coenzyme Q) with the above-mentioned reducing agent. Alternatively, fungus etc. containing reduced coenzyme Q can also be used. Furthermore, it is possible to reduce oxidized coenzyme Q into reduced coenzyme Q in a preparation by producing the preparation using oxidized coenzyme Q together with a substance having reducing ability such as vitamins and the like.

Preferred as reduced coenzyme Q to be used in the present invention is reduced coenzyme Q10 having 10 repeat structures in the side chain (the above-mentioned formula (1) wherein n is 10).

The cardiac dysfunction-ameliorating agent or cardiac function-maintaining agent of the present invention can also contain, besides reduced coenzyme Q, a known pharmaceutical agent for treating cardiac diseases. Such pharmaceutical agent for treating cardiac diseases is not particularly limited, and cardiotonic agent, antiarrhythmic agent, vasodilator, antihypertensive agent, antiplatelet agent, diuretic and the like, which are generally employed, can be used preferably. Specific examples of such pharmaceutical agent for treating cardiac diseases include digitoxin, digoxin, methyldigoxin, lanatoside C, proscillaridin, deslanoside, dopamine, doputamin, docarpamine, denopamine, aminophylline, amrinone, olprinone, milrinone, vesnarinone, pimobendan, bucladesine sodium, ubidecarenone, quinidine, procainamide, ajmaline, disopyramide, cibenzoline, lidocain, phenytoin, mexiletine, aprindine, flecainide, propafenone, pilsicainide, sotalol, amyl nitrite, nitroglycerol, isosorbide, diltiazem, nicardipine, nifedipine, verapamil, dilazep, dipyridamole, etafenone, trimetazidine, trapidil, nicorandil or a salt thereof and the like. While the proportion of reduced coenzyme Q and the above-mentioned pharmaceutical agent for treating cardiac diseases is not particularly limited, it is within the range of 100:1-1:100, preferably 10:1-1:10, by weight ratio.

In addition, the cardiac dysfunction-ameliorating agent or cardiac function-maintaining agent of the present invention can be used as it is, or in the form of a composition containing the same, which is used as a pharmaceutical product, a quasi-drug, a food, An animal drug, a feed and the like aiming at ameliorating cardiac dysfunction or maintaining cardiac function. In this case, the above-mentioned composition can contain various additives which are acceptable medically or under food hygiene law and the like. Such additives are not particularly limited and, for example, fats and oils, excipient, disintegrant, lubricant, binder, coating agent, colorant, anticoagulant, absorption promoter, solubilizing agents, stabilizer, health food material, dietary supplement material (supplement material) and the like can be mentioned.

The above-mentioned fats and oils are not particularly limited and edible fats and oils can be used. For example, plant oils such as corn oil, rape seed oil, soybean oil, sesame oil, olive oil, safflower oil, cottonseed oil, sunflower oil, rice bran oil, Japanese basil oil, perilla oil, flaxseed oil, tuberose oil, cacao butter, peanuts oil, palm oil, palm kernel oil and the like, animal oils such as fish oil, beef fat, lard, milk fat, egg-yolk oil and the like, synthetic oils such as medium-chain triglyceride and the like, fats and oils resulting from partitioning, hydrogenation, transesterification and the like of the above oils as starting materials, or a mixed oil thereof and the like can be mentioned.

The above-mentioned excipient is not particularly limited and, for example, sucrose, lactose, glucose, cornstarch, mannitol, crystalline cellulose, calcium phosphate, calcium sulfate and the like can be mentioned.

The above-mentioned disintegrant is not particularly limited and, for example, starch, agar, calcium citrate, calcium carbonate, sodium hydrogen carbonate, dextrin, crystalline cellulose, carboxymethylcellulose, tragacanth and the like can be mentioned.

The above-mentioned lubricant is not particularly limited and, for example, talc, magnesium stearate, polyethylene glycol, silica, hydrogenated vegetable oil and the like can be mentioned.

The above-mentioned binder is not particularly limited and, for example, ethylcellulose, methylcellulose, hydroxypropylmethylcellulose, tragacanth, shellac, gelatin, gum arabic, polyvinylpyrrolidone, polyvinyl alcohol, polyacrylic acid, polymethacrylic acid, sorbitol and the like can be mentioned.

The above-mentioned coating agent is not particularly limited, and gum arabic, opadry, asiatica, caster wax, carboxyvinyl polymer, carmellose, hydrated silicon dioxide, magnesium silicate, vinyl acetate resin, stearic acid, cetanol, hydroxypropylmethylcellulose and the like can be mentioned.

The above-mentioned colorant is not particularly limited and, for example, those permitted for addition to pharmaceutical products and food, and the like can be used.

The above-mentioned anticoagulant is not particularly limited and, for example, stearic acid, talc, light anhydrous silicic acid, hydrated silicon dioxide and the like can be mentioned.

The above-mentioned absorption promoter is not particularly limited and, for example, higher alcohols, higher fatty acids, surfactants such as glycerolfatty acid ester and the like can be mentioned.

The above-mentioned solubilizing agent is not particularly limited and, for example, organic acids such as fumaric acid, succinic acid, malic acid and the like can be mentioned.

The above-mentioned stabilizer is not particularly limited and, for example, benzoic acid, sodium benzoate, ethyl parahydroxybenzoate and the like can be mentioned.

The above-mentioned health food material is not particularly limited and, for example, kanpo medicines (e.g., ireito, unkeito, unsei'in, ogi-kentyuto, oren-gedokuto, orento, kakkonto, kami-kihito, kami-syoyosan, kanbaku-daisoto, kikyoto, kihito, kyumi-binroto, keigai-rengyoto, keisi-ka-syakuyaku-daioto, keihi-ka-syakuyakuto, keihi-ka-ryukotu-boreito, keisito, keisi-ninzinto, keisi-bukuryogan, keihito, kososan, gokoto, gosyakusan, gosha-jinkigan, gorinsan, saikanto, saiko-ka-ryukotu-boreito, saiko-keisi-kankyoto, saiko-keisito, saiko-seikanto, saibokuto, saireito, sansoninto, ziin-kokato, sigyakusan, sikunsito, simotuto, sya-kanzoto, syakuyakukanzoto, zyuzen-taihoto, zyumi-haidokuto, syoken-tyuto, syosaikoto, syoseiryuto, syohusan, sin'i-seihaito, sinpito, sinbuto, seizyo-bohuto, seisyo-ekkito, seisin-rensiin, seihaito, sokei-kakketuto, daio-kanzoto, daio-botanpito, daikentyuto, daisaikoto, daisaikoto-kyo-daio, daijyokito, daibohuto, jidaboku-ippo, tyoi-zyokito, tyotosan, tyoyoto, tyoreito, tyoreito-go-simotuto, tudosan, tokaku-zyokito, toki-insi, toki-kentyuto, toki-syakuyakusan, tokito, nitinto, nyosinsan, ninzinto, ninzin-yoeito, hainosankyuto, bakumondoto, hatimi-ziogan, hange-kobokuto, hange-syasinto, byakko-ka-ninzinto, bukuryoin, bukuryoin-go-hange-kobokuto, heiisan, boi-ogito, bohu-tusyosan, hotyu-ekkito, maoto, mao-busi-saisinto, makyo-kansekito, masiningan, mokuboito, yokukansan, yokukansan-ka-tinpi-hange, rikkunsito, rikkosan, ryutan-syakanto, ryokankyo-mi-singeninto, rokumi-ziogan and the like), tea leaves (e.g., green tea, unmilled rice tea, powdered tea, green tea of middle grade, toasted tea, roasted tea, jasmine tea, oolong tea, hongcha, heicha, huacha, jincha, baicha and the like), herbs (e.g., Italian parsley, elecampane, olive, oregano, cardoon, chamomile, curry plant, catnip, caraway, Christmas rose, crimson clover, cornflower, common mallow, salad burnet, santolina, cinnamon, jasmine, stevia, sage, European linden, scented geranium, St. John's wort, soapwort, Solomon's-seal, thyme, tansy, chervil, chive, nasturtium, jujube, basil, honeysuckle, hyssop, flax, fennel, foxglove, black hollyhock, French marigold, betony, heliotrope, bergamot, hemp agrimony, rue, pot marigold, borage, white horehound, myrtle, mullein, marjoram, mint, yarrow, lavender, lady's bedstraw, lemon grass, lemon verbena, lemon balm, rose, rosemary, rocket, wild strawberry, wild pansy, forget-me-not and the like), pycnogenol, flavangenol, propolis, ginkgo leaf, royal jelly, carnitine, mushrooms, aojiru (green-leaved-vegetable juice) and extract thereof and the like can be mentioned.

The above-mentioned nutritional supplementary food material is not particularly limited and amino acids, metal ions, proteins, saccharides, fatty acids, yeast extract, vegetable extract, fish meat extract, fruit, fruit extract and the like can be mentioned.

In addition, the cardiac dysfunction-ameliorating agent or cardiac function-maintaining agent of the present invention, or a composition containing the same can also contain other antioxidant substances, antioxidant enzyme, vitamins and the like. The antioxidant substance is not particularly limited and, for example, citric acid and derivatives thereof, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, pyrroloquinoline quinone and derivatives thereof and the like can be mentioned. The antioxidant enzyme is not particularly limited and, for example, superoxide dismutase (SOD), glutathione peroxidase, glutathione-S-transferase, glutathione reductase, catalases, ascorbic acid peroxidase and the like can be mentioned. The above-mentioned vitamins are not particularly limited and, for example, vitamin A, vitamin B, vitamin C, vitamin D, vitamin E, vitamin K or derivatives thereof and the like can be mentioned.

In the cardiac dysfunction-ameliorating agent or cardiac function-maintaining agent of the present invention, and a pharmaceutical product, a quasi-drug, a food, an animal drug and a feed, which contain the agent, the content, dosage form, preservation method and preservation form of reduced coenzyme Q are not limited, and can be appropriately determined according to the use thereof and product concept thereof. For example, in the cardiac dysfunction-ameliorating agent or cardiac function-maintaining agent of the present invention, and the above-mentioned composition containing the same, the content of reduced coenzyme Q is preferably 0.01-99 wt%, more preferably 0.1-50 wt%, relative to the whole preparation or composition.

The administration form and dosage form of the cardiac dysfunction-ameliorating agent or cardiac function-maintaining agent of the present invention, and a composition containing the same may be any form of liquid or solid, and various administration methods such as oral, injection, nasal, instillation, suppository, food containing reduced coenzyme Q, and the like can be employed. While oral administration is generally considered to be most effective from the aspects of dose and the like, when oral administration is difficult, the cardiac dysfunction-ameliorating agent or cardiac function-maintaining agent of the present invention and a composition containing the same can be administered by a route other than oral administration without any problem. For example, suppository, skin external preparation and the like may be, nonlimitatively, employed for patients or the elderly having difficulty in ingesting nutrients orally.

Examples of the form for preparing a composition containing the cardiac dysfunction-ameliorating agent or cardiac function-maintaining agent of the present invention as a pharmaceutical product or a quasi-drug include oral preparations (syrup, capsule, granule, pill, powder, tablet, drink), injection, infusion, nasal drop, eye drop, suppository and spray, as well as administration from the skin by ointment or an adhesive preparation.

The form of food containing the cardiac dysfunction-ameliorating agent or cardiac function-maintaining agent of the present invention is not particularly limited and, for example, general food forms such as edible fat and oil composition, cooking oil, spray oil, butter, margarine, shortening, whipping cream, concentrated milk, whiteners, dressings, pickle liquids, breads, cakes, pies, cookies, Japanese confectionaries, snacks, fried snacks, chocolates and chocolate confectionaries, rice confectionaries, roux, sauce, basting, toppings, ice creams, noodles, bread mix, fried food, processed meat products, fish paste products, frozen food such as frozen entrees, frozen meat and frozen vegetables, rice, jam, cheese, cheese food, cheese-like food, chewing gums, candies, fermented milk, canned food, drinks and the like, as well as supplement forms such as capsule, tablet and the like, food with health claims such as food for specified health uses, food with nutrient function claims and the like, health food, dietary supplement can be prepared. In the case of the above-mentioned food with health claims and supplement, a label can be placed thereon to indicate its use for amelioration of cardiac dysfunction or maintenance of cardiac function.

Furthermore, for animal purposes, the agent can be ingested/administered in a form such as an animal drug, a feed and the like.

The dosage in the amount of reduced coenzyme Q of the cardiac dysfunction-ameliorating agent or cardiac function-maintaining agent of the present invention and the above-mentioned composition containing the same is preferably 30-1200 mg for a day for an adult. It is more preferably 50-800 mg, and most preferably 100-300 mg. For children other than adults and animals, the above-mentioned preferable dosage can be calculated based on the body weight.

However, the dosage varies depending on the above-mentioned preferable dose and dosage form of the preparation, and highly absorbable preparation can achieve the desired object with a low dosage.

The above-mentioned dose per day can be ingested at one time or in several times. One dose of the cardiac dysfunction-ameliorating agent or cardiac function-maintaining agent of the present invention and a composition containing the same can be packed as a single unit. For example, in the case of a pharmaceutical product, food with health claims and supplement, a packing form wherein the ingestion per 1 dose is a unit, and in the case of a drink, a packing form wherein the drink is packed in a bottle and the like for complete ingestion at one time, and the like can be mentioned.

As mentioned above, the cardiac dysfunction-ameliorating agent or cardiac function-maintaining agent containing reduced coenzyme Q as an active ingredient can control cardiac function by administration to a subject. The control of cardiac function in this case includes not only ameliorating or treating cardiac dysfunction by ingestion of the cardiac dysfunction-ameliorating agent or cardiac function-maintaining agent of the present invention and a composition containing the same as a pharmaceutical product, but also maintaining cardiac function or preventing cardiac dysfunction by daily ingestion thereof as food. For example, the method of controlling cardiac function of the present invention also includes ingestion (administration) of the cardiac dysfunction-ameliorating agent or cardiac function-maintaining agent of the present invention to a healthy human or healthy animal with a normal cardiac function at present but having a potential risk of cardiac dysfunction, thereby to maintain cardiac function and prevent the condition of cardiac dysfunction, and administering the cardiac dysfunction-ameliorating agent or cardiac function-maintaining agent of the present invention to a patient or animal patient under the condition of cardiac dysfunction or under medication with other drugs and other treatments, as a support of the treatment. In other words, the method of controlling cardiac function of the present invention also includes concurrent ingestion of a known pharmaceutical agent for treating cardiac diseases as mentioned above and the cardiac dysfunction-ameliorating agent or cardiac function-maintaining agent containing reduced coenzyme Q as an active ingredient.

Furthermore, the present invention also provides a commercial package containing the above-mentioned cardiac dysfunction-ameliorating agent or cardiac function-maintaining agent and a written matter (for example, instruction etc.) relating thereto, which describes that the cardiac dysfunction-ameliorating agent or cardiac function-maintaining agent can or should be used for controlling cardiac function.

### Examples

The present invention is explained in more detail in the following by referring to Examples, which are not to be construed as limitative. The purity of reduced coenzyme Q10 and oxidized coenzyme Q10, and the ratio (weight ratio) of reduced coenzyme Q10/oxidized coenzyme Q10 was calculated by the above-mentioned HPLC analysis.

### (Production Example 1) Production of reduced coenzyme Q10

Oxidized coenzyme Q10 (100 g, purity 99.4%) and L-ascorbic acid (60 g) were added to ethanol (1000 g), and the mixture was stirred at 78°C to perform a reduction reaction.
After 30 hr, the reaction mixture was cooled to 50°C, and ethanol (330 g) and water (70 g) were added thereto while maintaining the same temperature. The ethanol solution (containing 100 g of reduced coenzyme Q10) was cooled to 2°C at a cooling rate of 10°C/hr with stirring to give white slurry.
The obtained slurry was filtered under reduced pressure, and the wet crystals were washed with cold ethanol, cold water and cold ethanol in this order (the temperature of the cold solvent used for washing was 2°C). Furthermore, the wet crystals were dried under reduced pressure (20-40°C, 1-30 mmHg) to give white dry crystals (97 g, reduced coenzyme Q10/oxidized coenzyme Q10=99/1). All operations except drying under reduced pressure were performed under a nitrogen atmosphere.

### (Production Example 2) Production of reduced coenzyme Q10

Oxidized coenzyme Q10 (100 g) was dissolved in heptane (100 g, 25°C). An aqueous solution of sodium hyposulfite (100 g, purity not less than 75%) in water (1000 ml) was gradually added as a reducing agent with stirring, and the reduction reaction was performed at 25°C, pH 4-6. After 2 hr, the aqueous phase was removed from the reaction mixture, and the heptane phase was washed 6 times with deaerated saturated brine (1000 g). All the above operations were performed under a nitrogen atmosphere. The heptane phase was subjected to solvent substitution under reduced pressure to give 7%(w/w) ethanol solution of reduced coenzyme Q10 at 50°C (containing 100 g of reduced coenzyme Q10). Water (50 g) was added to the ethanol solution, and cooled to 2°C at a cooling rate of 10°C/hr with stirring to precipitate crystals. All operations were performed under a nitrogen atmosphere. The obtained slurry was filtered under reduced pressure, and wet crystals were washed with cold ethanol, cold water and cold ethanol in this order (the temperature of the cold solvent to be used for washing was 2°C). Furthermore, the wet crystals were dried under reduced pressure (20-40°C, 1-30 mmHg) to give white dry crystals (97 g, reduced coenzyme Q10/oxidized coenzyme Q10=99/1).

### (Example 1) (Effect on cardiac disease)

### <Experiment system>

Male SD rats (9-week-old, Japan SLC Inc.) were divided into four groups. Each rat was anesthetized with pentobarbital sodium (35-45 mg/kg, i.p.), and fixed at the dorsal position. A tracheal tube was orally inserted into the airway, artificial respiration was performed by an artificial respirator for small animals, and the medial wall of the thorax was opened to expose the heart. In three groups, the left anterior descending artery (LAD) was obstructed for 30 min with a surgical needle with a thread. At this time, an electrocardiogram was measured via an amplifier for electrocardiogram, and the presence or absence of occlusion was confirmed based on the changes in the ST electrical potential and cardiac muscle color. An ischemia-reperfusion model was prepared by reperfusing the blood flow after 30 min from the occlusion. Thereafter, in all groups, the thorax was closed, sutured, and disinfected with a veterinary Isodine solution.

The following samples were administered to each group for 1 week before the above-mentioned operation and 4 weeks after the operation (total 5 weeks).

### 1 (Sham group: N=3)

Group for which open chest surgery alone was performed without occlusion treatment, and corn oil was orally administered at 5 ml/kg per day

### 2 (Control group: N=8)

Group for which occlusion treatment was performed, and corn oil was orally administered at 5 ml/kg per day

### 3 (Oxidized coenzyme Q10 group: N=7)

Group for which occlusion treatment was performed, and oxidized coenzyme Q10 dissolved in corn oil was orally administered at a dosage of 30 mg/kg per day based on the amount of oxidized coenzyme Q10

### 4 (Reduced coenzyme Q10 group: N=8)

Group for which occlusion treatment was performed, and oxidized coenzyme Q10 (containing 1 wt% of oxidized coenzyme Q10) dissolved in corn oil was orally administered at a dosage of 30 mg/kg per day based on the amount of reduced coenzyme Q10

### <Cardiac function evaluation>

After measuring the body weight on the next day of the final administration, the rats were fixed at the dorsal position under pentobarbital sodium (25-35 mg/kg, i.p.) anesthesia, and a mirror catheter was inserted into the right carotid artery. After confirmation of the stable condition, the left ventricle internal pressure wave pattern was led to a biological electric amplifier to enlarge same, and the left ventricular end-diastolic pressure (LVEDP) was measured.

### <Anatomy>

After cardiac function evaluation, the animals were euthanized by dislocation of cervical spine under anesthesia, and the heart was removed. The right atrium weight, left atrium weight, right ventricle weight, left ventricle weight and overall weight of the heart (weight of both atria + weight of both ventricles) were measured. For evaluation, the body weight ratio of the right atrium weight, the body weight ratio of the left atrium weight, the body weight ratio of the right ventricle weight and the body weight ratio of the left ventricle weight were calculated.

Table 1 shows the results of cardiac function evaluation (left ventricle diastolic pressure), and Table 2 shows the results of heart weight (body weight ratio).

**[Table 1]**

| (cardiac function) mean±standard error | | |
|---|---|---|
| | | left ventricle end-diastolic pressure (mmHg) |
| 1. | sham group | 4.1±0.3** |
| 2. | control group | 7.7±0.5 |
| 3. | oxidized coenzyme Q10 group | 8.3±0.7 |
| 4. | reduced coenzyme Q10 group | 5.7±0.4* |

| | | |
|---|---|---|
| (*p<0.05, **p<0.01 t-test vs. control group) | | |

**[Table 2]**

| (body weight ratio of heart weight) average | | | | | |
|---|---|---|---|---|---|
| | | right atrium | left atrium | right ventricle | left ventricle |
| 1. | sham group | 0.110 | 0.068 | 0.454 | 1.714 |
| 2. | control group | 0.187 | 0.100 | 0.546 | 1.906 |
| 3. | oxidized coenzyme group Q10 | 0.135 | 0.081 | 0.498 | 1.823 |
| 4. | reduced coenzyme group Q10 | 0.146 | 0.089 | 0.508 | 1.879 |

From the results of Table 1, the ischemia reperfusion model (control group) showed increased ventricular end-diastolic pressure as compared to the sham group, since cardiac function decreased to cause accumulation of blood. In contrast, it has been clarified that the group administered with reduced coenzyme Q10 showed significant suppression of elevation of the end-diastolic pressure, thus ameliorating the cardiac function. Administration of oxidized coenzyme Q10 did not provide an ameliorating effect.

From the results of Table 2, the ischemia reperfusion model (control group) showed decreased cardiac function as compared to the sham group, causing congested blood circulation leading to edema, which increased the heart weight. In contrast, it has been clarified that the oxidized coenzyme Q10 group and the group administered with reduced coenzyme Q10 showed a suppressed increase in the heart weight in all parts, and edema was ameliorated.

From the above-mentioned results, oxidized coenzyme Q10 and reduced coenzyme Q10 were common in that they both have an ameliorating effect on edema caused by cardiac ischemia. However, reduced coenzyme Q10 clearly showed an ameliorating effect on decreased cardiac function, for which oxidized coenzyme Q10 failed to show effect. Hence, reduced coenzyme Q10 is considered to have a stronger cardiac function ameliorating effect than oxidized coenzyme Q10, or effective for broader pathology relating to cardiac function.

### (Example 2) (Absorbability evaluation)

Example 1 revealed that reduced coenzyme Q10 has a stronger cardiac function-ameliorating effect than oxidized coenzyme Q10. On the other hand, it has been disclosed that oral absorbability of coenzyme Q10 can be enhanced by the co-presence of reduced coenzyme Q10, as compared to oxidized coenzyme Q10 alone, and that the utilization of reduced coenzyme Q10 is extremely effective for increasing oral absorbability in various uses (JP-A-10-109933). Thus, whether or not the difference in the effect in Example 1 is caused by the difference in the transferability of coenzyme Q10 into the heart was examined.

### <Experiment system>

Male SD rats (5-week-old, Japan SLC Inc.) were each orally administered with soybean oil (control group), oxidized coenzyme Q10 (100 mg/kg) dissolved in soybean oil (oxidized coenzyme Q10 group) and reduced coenzyme Q10 (containing 1 wt% of oxidized coenzyme Q10; 100 mg/kg) dissolved in soybean oil (reduced coenzyme Q10 group) once a day for 4 consecutive weeks (each group N=3). After completion of the administration, blood samples were taken and the heart was removed, and the total amount of coenzyme Q10 (total amount of oxidized coenzyme Q10 and reduced coenzyme Q10) in plasma and heart was quantified by HPLC according to a conventional method.

The results are shown in Table 3.

**[Table 3]**

| (concentration average: µg/ml) | | |
|---|---|---|
| | plasma | heart |
| control group | 0.02 | 9.59 |
| oxidized coenzyme Q10 group | 0.35 | 9.69 |
| reduced coenzyme Q10 group | 0.39* | 9.33 |

| | | |
|---|---|---|
| (*p<0.05 t-test comparison with oxidized coenzyme Q10 group) | | |

As shown in Table 3, the concentration of coenzyme Q10 in plasma was higher in the reduced coenzyme Q10 administration group than in the oxidized coenzyme Q10 administration group, as conventionally reported. However, the coenzyme Q10 concentration in the heart was not different in any administration group. Therefrom the difference in the cardiac function-ameliorating effect between reduced coenzyme Q10 and oxidized coenzyme Q10 cannot be explained only by the difference in the concentration in plasma. Hence, the more superior cardiac function-ameliorating effect of reduced coenzyme Q10 is an unexpected result.

### (Formulation Example 1) (Powder)

Reduced coenzyme Q10 (containing 1 wt% of oxidized coenzyme Q10) was dissolved in propanol, and adsorbed to microcrystalline cellulose and dried under reduced pressure. This was mixed with cornstarch under a nitrogen stream to give a powder.

| | |
|---|---|
| reduced coenzyme Q10 | 9.9 parts by weight |
| oxidized coenzyme Q10 | 0.1 part by weight |
| microcrystalline cellulose | 40 parts by weight |
| cornstarch | 55 parts by weight. |

### (Formulation Example 2) (Capsule)

In the same manner as in Formulation Example 1, a powder with the following formulation was prepared, and filled in a gelatin capsule according to a conventional method. The capsule thus filled was sealed, packed under a nitrogen atmosphere, and refrigerated for preservation.

| | |
|---|---|
| reduced coenzyme Q10 | 19.8 parts by weight |
| oxidized coenzyme Q10 | 0.2 part by weight |
| microcrystalline cellulose | 40 parts by weight |
| cornstarch | 20 parts by weight |
| lactose | 65 parts by weight |
| magnesium stearate | 3 parts by weight |
| polyvinylpyrrolidone | 2 parts by weight. |

### (Formulation Example 3) (Soft capsule)

Corn oil was heated to 50°C, and reduced coenzyme Q10 (containing about 1 wt% of oxidized coenzyme Q10) melted at the same temperature was added and dissolved therein. This was prepared into a soft-capsule according to a conventional method.

| | |
|---|---|
| reduced coenzyme Q10 | 49.5 parts by weight |
| oxidized coenzyme Q10 | 0.5 part by weight |
| corn oil | 350 parts by weight. |

### (Formulation Example 4) (Tablet)

Reduced coenzyme Q10 (containing about 1 wt% of oxidized coenzyme Q10) was dissolved in propanol, adsorbed to microcrystalline cellulose and dried under reduced pressure. This was mixed with cornstarch, lactose, carboxymethylcellulose and magnesium stearate under a nitrogen atmosphere. Then, an aqueous solution of polyvinylpyrrolidone was added as a binder and the mixture was granulated according to a conventional method. This was mixed with talc as a lubricant and the mixture was compressed to give a tablet. The tablet was packed under a nitrogen atmosphere and refrigerated for preservation.

| | |
|---|---|
| reduced coenzyme Q10 | 19.8 parts by weight |
| oxidized coenzyme Q10 | 0.2 part by weight |
| cornstarch | 25 parts by weight |
| lactose | 15 parts by weight |
| calcium carboxymethylcellulose | 10 parts by weight |
| microcrystalline cellulose | 40 parts by weight |
| polyvinylpyrrolidone | 5 parts by weight |
| magnesium stearate | 3 parts by weight |
| talc | 10 parts by weight |

## Claims

1. A cardiac dysfunction-ameliorating agent or cardiac function-maintaining agent comprising a reduced coenzyme Q represented by the following formula (1): 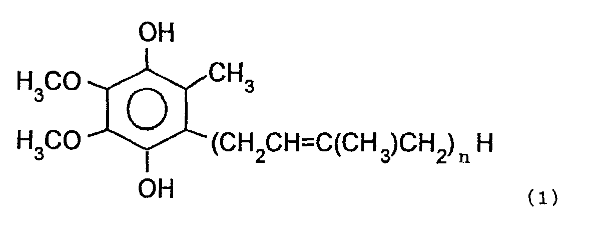 wherein n is an integer of 1-12, as an active ingredient.

2. The cardiac dysfunction-ameliorating agent or cardiac function-maintaining agent of claim 1, wherein the reduced coenzyme Q is a reduced coenzyme Q10 wherein n is 10.

3. The cardiac dysfunction-ameliorating agent or cardiac function-maintaining agent of claim 1 or 2, further comprising a pharmaceutical agent for treating a cardiac disease.

4. The cardiac dysfunction-ameliorating agent or cardiac function-maintaining agent of claim 3, wherein the pharmaceutical agent for treating a cardiac disease is one or more kinds of pharmaceutical agents selected from the group consisting of a cardiotonic agent, an antiarrhythmic agent, a vasodilator, an antihypertensive agent, an antiplatelet agent and a diuretic.

5. A pharmaceutical product or quasi-drug comprising the cardiac dysfunction-ameliorating agent or cardiac function-maintaining agent of any one of claims 1 to 4.

6. A food comprising the cardiac dysfunction-ameliorating agent or cardiac function-maintaining agent of any one of claims 1 to 4.

7. The food of claim 6, which is a food with health claims.

8. The food with health claims of claim 7, which is a food for specified health uses.

9. An animal drug comprising the cardiac dysfunction-ameliorating agent or cardiac function-maintaining agent of any one of claims 1 to 4.

10. A feed for ameliorating or preventing cardiac dysfunction, comprising the cardiac dysfunction-ameliorating agent or cardiac function-maintaining agent of any one of claims 1 to 4.

11. A method of controlling cardiac function, comprising administering a cardiac dysfunction-ameliorating agent or cardiac function-maintaining agent comprising a reduced coenzyme Q represented by the following formula (1): 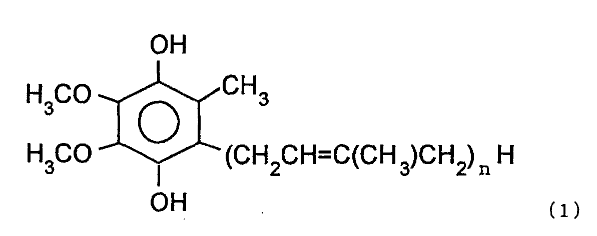 wherein n is an integer of 1-12, as an active ingredient, to a subject of administration.

12. The method of claim 11, wherein the subject of administration is a healthy human or healthy animal having a potential risk of cardiac dysfunction.

13. The method of claim 11, wherein the subject of administration is suffering from cardiac dysfunction and under a treatment thereof, and the method further comprises administering the cardiac dysfunction-ameliorating agent or cardiac function-maintaining agent as an aid for the treatment.

14. Use of a reduced coenzyme Q represented by the following formula (1): 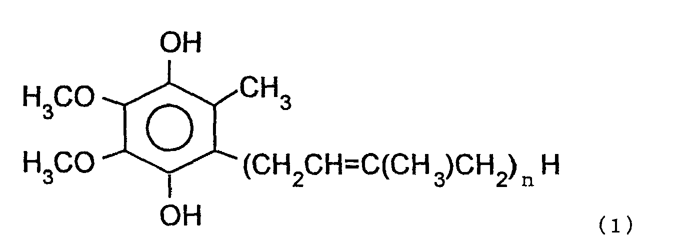 wherein n is an integer of 1-12, for the production of a cardiac dysfunction-ameliorating agent or cardiac function-maintaining agent.

15. A commercial package comprising the cardiac dysfunction-ameliorating agent or cardiac function-maintaining agent of any one of claims 1 to 4, and a written matter stating that the cardiac dysfunction-ameliorating agent or cardiac function-maintaining agent can or should be used for controlling cardiac function.
